# EUROPÄISCHE PATENTSCHRIFT

(11) **EP 2 731 975 B1**
(45) Veröffentlichungstag und Bekanntmachung des Hinweises auf die Patenterteilung: **20.04.2016**
(21) Anmeldenummer: 12733776.4
(22) Anmeldetag: 13.07.2012
(51) Int. Cl.: C08F 2/26

(54) **VERFAHREN ZUR HERSTELLUNG WASSERABSORBIERENDER POLYMERPARTIKEL MIT HOHER ANQUELLGESCHWINDIGKEIT**
METHOD FOR PRODUCING WATER-ABSORBING POLYMER PARTICLES HAVING A HIGH SWELLING SPEED
PROCÉDÉ DE PRÉPARATION DE PARTICULES POLYMÈRES ABSORBANT L'EAU AYANT UNE VITESSE DE GONFLEMENT ÉLEVÉE

(30) Priorität: 14.07.2011 EP 11173915
(43) Veröffentlichungstag der Anmeldung: 21.05.2014
(73) Patentinhaber: BASF SE, 67056 Ludwigshafen am Rhein (DE)
(72) Erfinder: BRAUN, Markus, 69118 Heidelberg (DE); WEISMANTEL, Matthias, 63637 Jossgrund (DE)
(74) Vertreter: BASF IP Association
(86) Internationale Anmeldenummer: PCT/EP2012/063801
(87) Internationale Veröffentlichungsnummer: WO 2013/007819

(56) Entgegenhaltungen:
- EP-A1- 1 236 781
- EP-A1- 2 267 038
- EP-A2- 1 760 095
- WO-A1-2011/023572
- WO-A1-2011/061315
- DE-A1-102006 030 557
- JP-A- H11 199 602
- JP-A- 2001 011 412

## Beschreibung

Die vorliegende Erfindung betrifft ein Verfahren zur Herstellung wasserabsorbierender Polymerpartikel mit hoher Anquellgeschwindigkeit durch Polymerisation einer Monomerlösung oder -suspension, enthaltend ein ethylenisch ungesättigtes, säuregruppentragendes Monomer, einen Vernetzer, einen Initiator und ein ethylenisch ungesättigtes, ionisches Tensid. Wasserabsorbierende Polymerpartikel werden zur Herstellung von Windeln, Tampons, Damenbinden und anderen Hygieneartikeln, aber auch als wasserzurückhaltende Mittel im landwirtschaftlichen Gartenbau verwendet. Die wasserabsorbierenden Polymerpartikel werden auch als Superabsorber bezeichnet.

Die Herstellung wasserabsorbierender Polymerpartikel wird in der Monographie "Modern Superabsorbent Polymer Technology", F.L. Buchholz und A.T. Graham, Wiley-VCH, 1998, Seiten 71 bis 103, beschrieben.

Die Eigenschaften der wasserabsorbierenden Polymerpartikel können beispielsweise über die verwendete Vernetzermenge eingestellt werden. Mit steigender Vernetzermenge sinkt die Zentrifugenretentionskapazität (CRC) und die Absorption unter einem Druck von 21,0 g/cm² (AUL0.3psi) durchläuft ein Maximum.

Zur Verbesserung der Anwendungseigenschaften, wie beispielsweise Permeabilität des gequollenen Gelbetts (SFC) in der Windel und Absorption unter einem Druck von 49,2 g/cm² (AUL0.7psi), werden wasserabsorbierende Polymerpartikel im allgemeinen oberflächennachvernetzt. Dadurch steigt der Vernetzungsgrad der Partikeloberfläche, wodurch die Absorption unter einem Druck von 49,2 g/cm² (AUL0.7psi) und die Zentrifugenretentionskapazität (CRC) zumindest teilweise entkoppelt werden können. Diese Oberflächennachvernetzung kann in wässriger Gelphase durchgeführt werden. Vorzugsweise werden aber getrocknete, gemahlene und abgesiebte Polymerpartikel (Grundpolymer) an der Oberfläche mit einem Oberflächennachvernetzer beschichtet und thermisch oberflächennachvernetzt. Dazu geeignete Vernetzer sind Verbindungen, die mit mindestens zwei Carboxylatgruppen der wasserabsorbierenden Polymerpartikel kovalente Bindungen bilden können.

Die ältere Anmeldung mit dem Aktenzeichen WO2011/131526 lehrt die Verwendung von

Comonomeren zur Erhöhung der Anquellgeschwindigkeit.

WO 2011/061315 offenbart ein Verfahren zur Herstellung wasserabsorbierender Polymerpartikel durch Polymerisation einer aufgeschäumten wässrigen Monomerlösung oder -suspension. WO 2011/023572 offenbart ein Verfahren zur Herstellung wasserabsorbierender Polymerpartikel durch Polymerisation von Tropfen einer Monomerlösung.

Aufgabe der vorliegenden Erfindung war die Bereitstellung eines verbesserten Verfahrens zur Herstellung wasserabsorbierender Polymerpartikel, insbesondere von wasserabsorbierenden Polymerpartikeln mit hoher Anquellgeschwindigkeit.

Gelöst wurde die Aufgabe durch ein Verfahren zur Herstellung wasserabsorbierender Polymerpartikel durch Polymerisation einer Monomerlösung oder -suspension, enthaltend
a) ein ethylenisch ungesättigtes, säuregruppentragendes Monomer, das zumindest teilweise neutralisiert sein kann,
b) mindestens einen Vernetzer,
c) mindestens einen Initiator,
d) optional ein mit den unter a) genannten Monomeren copolymerisierbares ethylenisch ungesättigtes Monomer und
e) optional ein oder mehrere wasserlösliche Polymere,
dadurch gekennzeichnet, dass die Monomerlösung oder -suspension mindestens ein ethylenisch ungesättigtes, ionisches Tensid enthält, das ethylenisch ungesättigte, ionischen Tensid eine Verbindung der allgemeinen Formel (I) list.

Die erfindungsgemäß einzusetzenden ethylenisch ungesättigten, ionischen Tenside sind grenzflächenaktive Verbindungen, die die Oberflächenspannung von Wasser senken, vorzugsweise unter 70mN/m, besonders bevorzugt unter 68 mN/m, ganz bevorzugt unter 67 mN/m, jeweils gemessen bei 23°C als 0,103gew.-%ige Lösung in Wasser.

Ethylenisch ungesättigte, ionische Tenside weisen vorzugsweise eine ethylenisch ungesättigte Gruppe, einen unpolaren Abstandshalter und eine ionische Endgruppe auf, wobei anionische Endgruppen bevorzugt sind. Geeignete ethylenisch ungesättigte Gruppen sind beispielsweise Allylether-, Vinylether-, Acrylester- und Methacrylester-Gruppen. Ein geeigneter unpolarer Abstandshalter ist beispielsweise eine Polypropylenglykol-Gruppe. Geeignete ionische Endgruppen sind beispielsweise quartäre Amin-, Phosphat- und Sulfat-Gruppen.

Erfindungsgemäße Ungesättigte ionische Tenside sind Verbindungen der allgemeinen Formel (I) 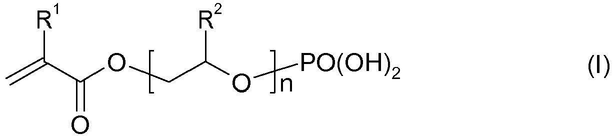 wobei
- R¹ und R²: unabhängig voneinander Wasserstoff, Methyl oder Ethyl, vorzugsweise Methyl oder Ethyl, ganz besonders bevorzugt Methyl, sind und
- n: eine ganze Zahl von 3 bis 20, vorzugsweise von 4 bis 15, ganz besonders bevorzugt von 5 bis 10, ist.

Die Monomerlösung oder -suspension enthält vorzugsweise 0,005 bis 1 Gew.-%, besonders bevorzugt von 0,02 bis 0,5 Gew.--%, ganz besonders bevorzugt von 0,05 bis 0,2 Gew.-%, ethylenisch ungesättigtes, ionisches Tensid, jeweils bezogen auf das unneutralisierte Monomer a).

Der vorliegenden Erfindung liegt die Erkenntnis zugrunde, dass bereits geringe Mengen an ethylenisch ungesättigten, ionischen Tensiden die Anquellgeschwindigkeit (FSR) deutlich erhöhen. Dadurch dass die ethylenisch ungesättigten, ionischen Tenside in das Polymernetzwerk eingebaut werden, ist ihr Einfluss auf die Oberflächenspannung des wässrigen Extrakts gering.

Die Menge an ethylenisch ungesättigtem, ionischem Tensid wird dabei üblicherweise so gewählt, dass die Oberflächenspannung des wässrigen Extrakts vorzugweise mindestens 55 mN/m, besonders bevorzugt mindesten 60 mN/m, ganz besonders bevorzugt mindestens 65 mN/m, beträgt.

Die Ursache dafür, dass bei Verwendung der erfindungsgemäß einzusetzenden ethylenisch ungesättigten, ionischen Tenside trotzdem die Oberflächenspannung des wässrigen Extrakts abnimmt dürfte darauf zurückzuführen sein, dass a) der Umsatz der ethylenisch ungesättigten, ionischen Tenside nicht vollständig ist und/oder b) dass die einpolymerisierten ethylenisch ungesättigten, ionischen Tenside nachträglich tensidische Gruppen, beispielsweise bei Verwendung von Acrylsäureestern und Methacrylsäureestern durch Esterhydrolyse, abspalten.

Die wasserabsorbierenden Polymerpartikel werden durch Polymerisation einer Monomerlösung oder -suspension hergestellt und sind üblicherweise wasserunlöslich.

Das Monomer a) ist vorzugsweise wasserlöslich, d.h. die Löslichkeit in Wasser bei 23°C beträgt typischerweise mindestens 1 g/100 g Wasser, vorzugsweise mindestens 5 g/100 g Wasser, besonders bevorzugt mindestens 25 g/100 g Wasser, ganz besonders bevorzugt mindestens 35 g/100 g Wasser.

Geeignete Monomere a) sind beispielsweise ethylenisch ungesättigte Carbonsäuren, wie Acrylsäure, Methacrylsäure, und Itaconsäure. Besonders bevorzugte Monomere a) sind Acrylsäure und Methacrylsäure. Ganz besonders bevorzugt ist Acrylsäure.

Weitere geeignete Monomere a) sind beispielsweise ethylenisch ungesättigte Sulfonsäuren, wie Styrolsulfonsäure und 2-Acrylamido-2-methylpropansulfonsäure (AMPS).

Verunreinigungen können einen erheblichen Einfluss auf die Polymerisation haben. Daher sollten die eingesetzten Rohstoffe eine möglichst hohe Reinheit aufweisen. Es ist daher oft vorteilhaft die Monomeren a) speziell zu reinigen. Geeignete Reinigungsverfahren werden beispielsweise in der WO 2002/055469 A1, der WO 2003/078378 A1 und der WO 2004/035514 A1 beschrieben. Ein geeignetes Monomer a) ist beispielsweise eine gemäß WO 2004/035514 A1 gereinigte Acrylsäure mit 99,8460 Gew.-% Acrylsäure, 0,0950 Gew.-% Essigsäure, 0,0332 Gew.-% Wasser, 0,0203 Gew.-% Propionsäure, 0,0001 Gew.-% Furfurale, 0,0001 Gew.-% Maleinsäureanhydrid, 0,0003 Gew.-% Diacrylsäure und 0,0050 Gew.-% Hydrochinonmonomethylether.

Das Monomer a) enthält üblicherweise Polymerisationsinhibitoren, vorzugsweise Hydrochinonhalbether, als Lagerstabilisator.

Die Monomerlösung enthält vorzugsweise bis zu 250 Gew.-ppm, bevorzugt höchstens 130 Gew.-ppm, besonders bevorzugt höchstens 70 Gew.-ppm, bevorzugt mindestens 10 Gew.-ppm, besonders bevorzugt mindestens 30 Gew.-ppm, insbesondere um 50 Gew.-ppm, Hydrochinonhalbether, jeweils bezogen auf das unneutralisierte Monomer a). Beispielsweise kann zur Herstellung der Monomerlösung ein ethylenisch ungesättigtes, säuregruppentragendes Monomer mit einem entsprechenden Gehalt an Hydrochinonhalbether verwendet werden.

Bevorzugte Hydrochinonhalbether sind Hydrochinonmonomethylether (MEHQ) und/oder alpha-Tocopherol (Vitamin E).

Geeignete Vernetzer b) sind Verbindungen mit mindestens zwei zur Vernetzung geeigneten Gruppen. Derartige Gruppen sind beispielsweise ethylenisch ungesättigte Gruppen, die in die Polymerkette radikalisch einpolymerisiert werden können, und funktionelle Gruppen, die mit den Säuregruppen des Monomeren a) kovalente Bindungen ausbilden können. Weiterhin sind auch polyvalente Metallsalze, die mit mindestens zwei Säuregruppen des Monomeren a) koordinative Bindungen ausbilden können, als Vernetzer b) geeignet.

Vernetzer b) sind vorzugsweise Verbindungen mit mindestens zwei polymerisierbaren Gruppen, die in das Polymernetzwerk radikalisch einpolymerisiert werden können. Geeignete Vernetzer b) sind beispielsweise Ethylenglykoldimethacrylat, Diethylenglykoldiacrylat, Polyethylenglykoldiacrylat, Allylmethacrylat, Trimethylolpropantriacrylat, Triallylamin, Tetraallylammoniumchlorid, Tetraallyloxyethan, wie in EP 0 530 438 A1 beschrieben, Di- und Triacrylate, wie in EP 0 547 847 A1, EP 0 559 476 A1, EP 0 632 068 A1, WO 93/21237 A1, WO 2003/104299 A1, WO 2003/104300 A1, WO 2003/104301 A1 und DE 103 31 450 A1 beschrieben, gemischte Acrylate, die neben Acrylatgruppen weitere ethylenisch ungesättigte Gruppen enthalten, wie in DE 103 31 456 A1 und DE 103 55 401 A1 beschrieben, oder Vernetzermischungen, wie beispielsweise in DE 195 43 368 A1, DE 196 46 484 A1, WO 90/15830 A1 und WO 2002/032962 A2 beschrieben.

Bevorzugte Vernetzer b) sind Pentaerythrittriallylether, Tetraallyloxyethan, Methylenbismethacrylamid, 15-fach ethoxyliertes Trimethylolpropantriacrylat, Polyethylenglykoldiacrylat, Trimethylolpropantriacrylat, Triallylamin und Tetraallylammoniumchlorid.

Ganz besonders bevorzugte Vernetzer b) sind die mit Acrylsäure oder Methacrylsäure zu Di- oder Triacrylaten veresterten mehrfach ethoxylierten und/oder propoxylierten Glyzerine, wie sie beispielsweise in WO 2003/104301 A1 beschrieben sind. Besonders vorteilhaft sind Di- und/oder Triacrylate des 3- bis 10-fach ethoxylierten Glyzerins. Ganz besonders bevorzugt sind Di- oder Triacrylate des 1- bis 5-fach ethoxylierten und/oder propoxylierten Glyzerins. Am meisten bevorzugt sind die Triacrylate des 3- bis 5-fach ethoxylierten und/oder propoxylierten Glyzerins, insbesondere das Triacrylat des 3-fach ethoxylierten Glyzerins.

Die Menge an Vernetzer b) beträgt vorzugsweise 0,05 bis 1,5 Gew.-%, besonders bevorzugt 0,1 bis 1 Gew.-%, ganz besonders bevorzugt 0,2 bis 0,5 Gew.-%, jeweils bezogen auf das unneutralisierte Monomer a). Mit steigendem Vernetzergehalt sinkt die Zentrifugenretentionskapazität (CRC) und die Absorption unter einem Druck von 21,0 g/cm² (AUL0.3psi) durchläuft ein Maximum.

Als Initiatoren c) können sämtliche unter den Polymerisationsbedingungen Radikale erzeugende Verbindungen eingesetzt werden, beispielsweise thermische Initiatoren, Redox-Initiatoren, Photoinitiatoren. Geeignete Redox-Initiatoren sind Natriumperoxodisulfat/Ascorbinsäure, Wasserstoffperoxid/Ascorbinsäure, Natriumperoxodisulfat/Natriumbisulfit und Wasserstoffperoxid/Natriumbisulfit. Vorzugsweise werden Mischungen aus thermischen Initiatoren und Redox-Initiatoren eingesetzt, wie Natriumperoxodisulfat/Wasserstoffperoxid/Ascorbinsäure. Als reduzierende Komponente wird aber vorzugsweise ein Gemisch aus dem Dinatriumsalz der 2-Hydroxy-2-sulfinatoessigsäure, dem Dinatriumsalz der 2-Hydroxy-2-sulfonatoessigsäure und Natriumbisulfit (erhältlich als Brüggolit® FF6 und Brüggolit® FF7 von Brüggemann Chemicals; Heilbronn; DE) oder das Dinatriumsalz der 2-Hydroxy-2-sulfinatoessigsäure in Reinform (erhältlich als Blancolen® HP von Brüggemann Chemicals; Heilbronn; DE), eingesetzt.

Mit den ethylenisch ungesättigten, säuregruppentragenden Monomeren a) copolymerisierbare ethylenisch ungesättigte Monomere d) sind beispielsweise Acrylamid, Methacrylamid, Hydroxyethylacrylat, Hydroxyethylmethacrylat, Dimethylaminoethylmethacrylat, Dimethylaminoethylacrylat, Dimethylaminopropylacrylat, Diethylaminopropylacrylat, Dimethylaminoethylmethacrylat, Diethylaminoethylmethacrylat.

Als wasserlösliche Polymere e) können Polyvinylalkohol, Polyvinylpyrrolidon, Stärke, Stärkederivate, modifizierte Cellulose, wie Methylcellulose oder Hydroxyethylcellulose, Gelatine, Polyglykole oder Polyacrylsäuren, vorzugsweise Stärke, Stärkederivate und modifizierte Cellulose, eingesetzt werden.

Üblicherweise wird eine wässrige Monomerlösung verwendet. Der Wassergehalt der Monomerlösung beträgt vorzugsweise von 40 bis 75 Gew.-%, besonders bevorzugt von 45 bis 70 Gew.-%, ganz besonders bevorzugt von 50 bis 65 Gew.-%. Es ist auch möglich Monomersuspensionen, d.h. Monomerlösungen mit überschüssigem Monomer a), beispielsweise Natriumacrylat, einzusetzen. Mit steigendem Wassergehalt steigt der Energieaufwand bei der anschließenden Trocknung und mit sinkendem Wassergehalt kann die Polymerisationswärme nur noch ungenügend abgeführt werden.

Die bevorzugten Polymerisationsinhibitoren benötigen für eine optimale Wirkung gelösten Sauerstoff. Daher kann die Monomerlösung vor der Polymerisation durch Inertisierung, d.h. Durchströmen mit einem inerten Gas, vorzugsweise Stickstoff oder Kohlendioxid, von gelöstem Sauerstoff befreit werden. Vorzugsweise wird der Sauerstoffgehalt der Monomerlösung vor der Polymerisation auf weniger als 1 Gew.-ppm, besonders bevorzugt auf weniger als 0,5 Gew.-ppm, ganz besonders bevorzugt auf weniger als 0,1 Gew.-ppm, gesenkt.

Geeignete Reaktoren sind beispielsweise Knetreaktoren oder Bandreaktoren. Im Kneter wird das bei der Polymerisation einer wässrigen Monomerlösung oder -suspension entstehende Polymergel durch beispielsweise gegenläufige Rührwellen kontinuierlich zerkleinert, wie in WO 2001/038402 A1 beschrieben. Die Polymerisation auf dem Band wird beispielsweise in DE 38 25 366 A1 und US 6,241,928 beschrieben. Bei der Polymerisation in einem Bandreaktor entsteht ein Polymergel, das in einem weiteren Verfahrensschritt zerkleinert werden muss, beispielsweise in einem Extruder oder Kneter.

Zur Verbesserung der Trocknungseigenschaften kann das mittels eines Kneters erhaltene zerkleinerte Polymergel zusätzlich extrudiert werden.

Es ist aber auch möglich eine wässrige Monomerlösung zu vertropfen und die erzeugten Tropfen in einem erwärmten Trägergasstrom zu polymerisieren. Hierbei können die Verfahrensschritte Polymerisation und Trocknung zusammengefasst werden, wie in WO 2008/040715 A2, WO 2008/052971 A1 und WO 2011/026876 A1 beschrieben.

Die Säuregruppen der erhaltenen Polymergele sind üblicherweise teilweise neutralisiert. Die Neutralisation wird vorzugsweise auf der Stufe der Monomeren durchgeführt. Dies geschieht üblicherweise durch Einmischung des Neutralisationsmittels als wässrige Lösung oder bevorzugt auch als Feststoff. Der Neutralisationsgrad beträgt vorzugsweise von 25 bis 95 mol-%, besonders bevorzugt von 30 bis 80 mol-%, ganz besonders bevorzugt von 40 bis 75 mol-%, wobei die üblichen Neutralisationsmittel verwendet werden können, vorzugsweise Alkalimetallhydroxide, Alkalimetalloxide, Alkalimetallkarbonate oder Alkalimetallhydrogenkarbonate sowie deren Mischungen. Statt Alkalimetallsalzen können auch Ammoniumsalze verwendet werden. Natrium und Kalium sind als Alkalimetalle besonders bevorzugt, ganz besonders bevorzugt sind jedoch Natriumhydroxid, Natriumkarbonat oder Natriumhydrogenkarbonat sowie deren Mischungen.

Es ist aber auch möglich die Neutralisation nach der Polymerisation auf der Stufe des bei der Polymerisation entstehenden Polymergels durchzuführen. Weiterhin ist es möglich bis zu 40 mol-%, vorzugsweise 10 bis 30 mol-%, besonders bevorzugt 15 bis 25 mol-%, der Säuregruppen vor der Polymerisation zu neutralisieren indem ein Teil des Neutralisationsmittels bereits der Monomerlösung zugesetzt und der gewünschte Endneutralisationsgrad erst nach der Polymerisation auf der Stufe des Polymergels eingestellt wird. Wird das Polymergel zumindest teilweise nach der Polymerisation neutralisiert, so wird das Polymergel vorzugsweise mechanisch zerkleinert, beispielsweise mittels eines Extruders, wobei das Neutralisationsmittel aufgesprüht, übergestreut oder aufgegossen und dann sorgfältig untergemischt werden kann. Dazu kann die erhaltene Gelmasse noch mehrmals zur Homogenisierung extrudiert werden.

Das Polymergel wird dann vorzugsweise mit einem Bandtrockner getrocknet bis der Restfeuchtegehalt vorzugsweise 0,5 bis 15 Gew.-%, besonders bevorzugt 1 bis 10 Gew.-%, ganz besonders bevorzugt 2 bis 8 Gew.-%, beträgt, wobei der Restfeuchtegehalt gemäß der von der EDANA empfohlenen Testmethode Nr. WSP 230.2-05 "Mass Loss Upon Heating" bestimmt wird. Bei einer zu hohen Restfeuchte weist das getrocknete Polymergel eine zu niedrige Glasübergangstemperatur T_{g} auf und ist nur schwierig weiter zu verarbeiten. Bei einer zu niedrigen Restfeuchte ist das getrocknete Polymergel zu spröde und in den anschließenden Zerkleinerungsschritten fallen unerwünscht große Mengen an Polymerpartikeln mit zu niedriger Partikelgröße ("fines") an. Der Feststoffgehalt des Gels beträgt vor der Trocknung vorzugsweise von 25 und 90 Gew.-%, besonders bevorzugt von 35 bis 70 Gew.-%, ganz besonders bevorzugt von 40 bis 60 Gew.-%. Wahlweise kann zur Trocknung aber auch ein Wirbelbetttrockner oder ein Schaufeltrockner verwendet werden.

Das getrocknete Polymergel wird hiernach gemahlen und klassiert, wobei zur Mahlung üblicherweise ein- oder mehrstufige Walzenstühle, bevorzugt zwei- oder dreistufige Walzenstühle, Stiftmühlen, Hammermühlen oder Schwingmühlen, eingesetzt werden können.

Die mittlere Partikelgröße der als Produktfraktion abgetrennten Polymerpartikel beträgt vorzugsweise mindestens 200 µm, besonders bevorzugt von 250 bis 600 µm, ganz besonders von 300 bis 500 µm. Die mittlere Partikelgröße der Produktfraktion kann mittels der von der EDANA empfohlenen Testmethode Nr. WSP 220.2-05 "Partikel Size Distribution" ermittelt werden, wobei die Massenanteile der Siebfraktionen kumuliert aufgetragen werden und die mittlere Partikelgröße graphisch bestimmt wird. Die mittlere Partikelgröße ist hierbei der Wert der Maschenweite, der sich für kumulierte 50 Gew.-% ergibt.

Der Anteil an Partikeln mit einer Partikelgröße von mindestens 150 µm beträgt vorzugsweise mindestens 90 Gew.-%, besonders bevorzugt mindesten 95 Gew.-%, ganz besonders bevorzugt mindestens 98 Gew.-%.

Polymerpartikel mit zu niedriger Partikelgröße senken die Permeabilität (SFC). Daher sollte der Anteil zu kleiner Polymerpartikel ("fines") niedrig sein.

Zu kleine Polymerpartikel werden daher üblicherweise abgetrennt und in das Verfahren rückgeführt. Dies geschieht vorzugsweise vor, während oder unmittelbar nach der Polymerisation, d.h. vor der Trocknung des Polymergels. Die zu kleinen Polymerpartikel können vor oder während der Rückführung mit Wasser und/oder wässrigem Tensid angefeuchtet werden.

Es ist auch möglich in späteren Verfahrensschritten zu kleine Polymerpartikel abzutrennen, beispielsweise nach der Oberflächennachvernetzung oder einem anderen Beschichtungsschritt. In diesem Fall sind die rückgeführten zu kleinen Polymerpartikel oberflächennachvernetzt bzw. anderweitig beschichtet, beispielsweise mit pyrogener Kieselsäure.

Wird zur Polymerisation ein Knetreaktor verwendet, so werden die zu kleinen Polymerpartikel vorzugsweise während des letzten Drittels der Polymerisation zugesetzt.

Werden die zu kleinen Polymerpartikel sehr spät zugesetzt, beispielsweise erst in einem dem Polymerisationsreaktor nachgeschalteten Apparat, beispielsweise einem Extruder, so lassen sich die zu kleinen Polymerpartikel nur noch schwer in das erhaltene Polymergel einarbeiten. Unzureichend eingearbeitete zu kleine Polymerpartikel lösen sich aber während der Mahlung wieder von dem getrockneten Polymergel, werden beim Klassieren daher erneut abgetrennt und erhöhen die Menge rückzuführender zu kleiner Polymerpartikel.

Der Anteil an Partikeln mit einer Partikelgröße von höchstens 850 µm, beträgt vorzugsweise mindestens 90 Gew.-%, besonders bevorzugt mindesten 95 Gew.-%, ganz besonders bevorzugt mindestens 98 Gew.-%.

Der Anteil an Partikeln mit einer Partikelgröße von höchstens 600 µm, beträgt vorzugsweise mindestens 90 Gew.-%, besonders bevorzugt mindesten 95 Gew.-%, ganz besonders bevorzugt mindestens 98 Gew.-%.

Polymerpartikel mit zu großer Partikelgröße senken die Anquellgeschwindigkeit. Daher sollte der Anteil zu großer Polymerpartikel ebenfalls niedrig sein.

Zu große Polymerpartikel werden daher üblicherweise abgetrennt und in die Mahlung des getrockneten Polymergels rückgeführt.

Die Polymerpartikel können zur weiteren Verbesserung der Eigenschaften oberflächennachvernetzt werden. Geeignete Oberflächennachvernetzer sind Verbindungen, die Gruppen enthalten, die mit mindestens zwei Carboxylatgruppen der Polymerpartikel kovalente Bindungen bilden können. Geeignete Verbindungen sind beispielsweise polyfunktionelle Amine, polyfunktionelle Amidoamine, polyfunktionelle Epoxide, wie in EP 0 083 022 A2, EP 0 543 303 A1 und EP 0 937 736 A2 beschrieben, di- oder polyfunktionelle Alkohole, wie in DE 33 14 019 A1, DE 35 23 617 A1 und EP 0 450 922 A2 beschrieben, oder ß-Hydroxyalkylamide, wie in DE 102 04 938 A1 und US 6,239,230 beschrieben.

Des weiteren sind in DE 40 20 780 C1 zyklische Karbonate, in DE 198 07 502 A1 2-Oxazolidinon und dessen Derivate, wie 2-Hydroxyethyl-2-oxazolidinon, in DE 198 07 992 C1 Bis- und Poly-2-oxazolidinone, in DE 198 54 573 A1 2-Oxotetrahydro-1,3-oxazin und dessen Derivate, in DE 198 54 574 A1 N-Acyl-2-Oxazolidinone, in DE 102 04 937 A1 zyklische Harnstoffe, in DE 103 34 584 A1 bizyklische Amidoacetale, in EP 1 199 327 A2 Oxetane und zyklische Harnstoffe und in WO 2003/031482 A1 Morpholin-2,3-dion und dessen Derivate als geeignete Oberflächennachvernetzer beschrieben.

Bevorzugte Oberflächennachvernetzer sind Ethylenkarbonat, Ethylenglykoldiglycidylether, Umsetzungsprodukte von Polyamiden mit Epichlorhydrin und Gemische aus Propylenglykol und 1,4-Butandiol.

Ganz besonders bevorzugte Oberflächennachvernetzer sind 2-Hydroxyethyl-2-oxazolidinon, 2-Oxazolidinon und 1,3-Propandiol.

Weiterhin können auch Oberflächennachvernetzer eingesetzt werden, die zusätzliche polymerisierbare ethylenisch ungesättigte Gruppen enthalten, wie in DE 37 13 601 A1 beschrieben

Die Menge an Oberflächennachvernetzer beträgt vorzugsweise 0,001 bis 5 Gew.-%, besonders bevorzugt 0,02 bis 2 Gew.-%, ganz besonders bevorzugt 0,05 bis 1 Gew.-%, jeweils bezogen auf die Polymerpartikel.

In einer bevorzugten Ausführungsform der vorliegenden Erfindung werden vor, während oder nach der Oberflächennachvernetzung zusätzlich zu den Oberflächennachvernetzern polyvalente Kationen auf die Partikeloberfläche aufgebracht.

Die im erfindungsgemäßen Verfahren einsetzbaren polyvalenten Kationen sind beispielsweise zweiwertige Kationen, wie die Kationen von Zink, Magnesium, Kalzium, Eisen und Strontium, dreiwertige Kationen, wie die Kationen von Aluminium, Eisen, Chrom, Seltenerden und Mangan, vierwertige Kationen, wie die Kationen von Titan und Zirkonium. Als Gegenion sind Hydroxid, Chlorid, Bromid, Sulfat, Hydrogensulfat, Carbonat, Hydrogencarbonat, Nitrat, Phosphat, Hydrogenphosphat, Dihydrogenphosphat und Carboxylat, wie Acetat, Citrat und Lactat, möglich. Es sind auch Salze mit unterschiedlichen Gegenionen möglich, beispielsweise basische Aluminiumsalze, wie Aluminiummonoacetat oder Aluminiummonolaktat. Aluminiumsulfat, Aluminiummonoacetat und Aluminiumlaktat sind bevorzugt. Außer Metallsalzen können auch Polyamine als polyvalente Kationen eingesetzt werden.

Die Einsatzmenge an polyvalentem Kation beträgt beispielsweise 0,001 bis 1 Gew.-%, vorzugsweise 0,005 bis 0,5 Gew.-%, besonders bevorzugt 0,02 bis 0,2 Gew.-%. jeweils bezogen auf die Polymerpartikel.

Die Oberflächennachvernetzung wird üblicherweise so durchgeführt, dass eine Lösung des Oberflächennachvernetzers auf die getrockneten Polymerpartikel aufgesprüht wird. Im Anschluss an das Aufsprühen werden die mit Oberflächennachvernetzer beschichteten Polymerpartikel thermisch getrocknet, wobei die Oberflächennachvernetzungsreaktion sowohl vor als auch während der Trocknung stattfinden kann.

Das Aufsprühen einer Lösung des Oberflächennachvernetzers wird vorzugsweise in Mischern mit bewegten Mischwerkzeugen, wie Schneckenmischer, Scheibenmischer und Schaufelmischer, durchgeführt werden. Besonders bevorzugt sind Horizontalmischer, wie Schaufelmischer, ganz besonders bevorzugt sind Vertikalmischer. Die Unterscheidung in Horizontalmischer und Vertikalmischer erfolgt über die Lagerung der Mischwelle, d.h. Horizontalmischer haben eine horizontal gelagerte Mischwelle und Vertikalmischer haben eine vertikal gelagerte Mischwelle. Geeignete Mischer sind beispielsweise Horizontale Pflugschar® Mischer (Gebr. Lödige Maschinenbau GmbH; Paderborn; Deutschland), Vrieco-Nauta Continuous Mixer (Hosokawa Micron BV; Doetinchem; Niederlande), Processall Mixmill Mixer (Processall Incorporated; Cincinnati; U.S.A.) und Schugi Flexomix® (Hosokawa Micron BV; Doetinchem; Niederlande). Es ist aber auch möglich die Oberflächennachvernetzerlösung in einem Wirbelbett aufzusprühen.

Die Oberflächennachvernetzer werden typischerweise als wässrige Lösung eingesetzt. Über den Gehalt an nichtwässrigem Lösungsmittel bzw. Gesamtlösungsmittelmenge kann die Eindringtiefe des Oberflächennachvernetzers in die Polymerpartikel eingestellt werden.

Vorzugsweise werden Lösungsmittelgemische eingesetzt, beispielsweise Isopropanol/Wasser, 1,3-Propandiol/Wasser und Propylenglykol/Wasser, wobei das Mischungsmassenverhältnis vorzugsweise von 20:80 bis 40:60 beträgt.

Die thermische Trocknung wird vorzugsweise in Kontakttrocknern, besonders bevorzugt Schaufeltrocknern, ganz besonders bevorzugt Scheibentrocknern, durchgeführt. Geeignete Trockner sind beispielsweise Hosokawa Bepex® Horizontal Paddle Dryer (Hosokawa Micron GmbH; Leingarten; Deutschland), Hosokawa Bepex® Disc Dryer (Hosokawa Micron GmbH; Leingarten; Deutschland), Holo-Flite® dryers (Metso Minerals Industries Inc.; Danville; U.S.A.) und Nara Paddle Dryer (NARA Machinery Europe; Frechen; Deutschland). Überdies können auch Wirbelschichttrockner eingesetzt werden.

Die Trocknung kann im Mischer selbst erfolgen, durch Beheizung des Mantels oder Einblasen von Warmluft. Ebenso geeignet ist ein nachgeschalteter Trockner, wie beispielsweise ein Hordentrockner, ein Drehrohrofen oder eine beheizbare Schnecke. Besonders vorteilhaft wird in einem Wirbelschichttrockner gemischt und getrocknet.

Bevorzugte Trocknungstemperaturen liegen im Bereich 100 bis 250°C, bevorzugt 120 bis 220°C, besonders bevorzugt 130 bis 210°C, ganz besonders bevorzugt 150 bis 200°C. Die bevorzugte Verweilzeit bei dieser Temperatur im Reaktionsmischer oder Trockner beträgt vorzugsweise mindestens 10 Minuten, besonders bevorzugt mindestens 20 Minuten, ganz besonders bevorzugt mindestens 30 Minuten, und üblicherweise höchstens 60 Minuten.

In einer bevorzugten Ausführungsform der vorliegenden Erfindung werden die wasserabsorbierenden Polymerpartikel nach der thermischen Trocknung gekühlt. Die Kühlung wird vorzugsweise in Kontaktkühlern, besonders bevorzugt Schaufelkühlern, ganz besonders bevorzugt Scheibenkühlern, durchgeführt. Geeignete Kühler sind beispielsweise Hosokawa Bepex® Horizontal Paddle Cooler (Hosokawa Micron GmbH; Leingarten; Deutschland), Hosokawa Bepex® Disc Cooler (Hosokawa Micron GmbH; Leingarten; Deutschland), Holo-Flite® coolers (Metso Minerals Industries Inc.; Danville; U.S.A.) und Nara Paddle Cooler (NARA Machinery Europe; Frechen; Deutschland). Überdies können auch Wirbelschichtkühler eingesetzt werden.

Im Kühler werden die wasserabsorbierenden Polymerpartikel auf 20 bis 150°C, vorzugsweise 40 bis 120°C, besonders bevorzugt 60 bis 100°C, ganz besonders bevorzugt 70 bis 90°C, abgekühlt.

Anschließend können die oberflächennachvernetzten Polymerpartikel erneut klassiert werden, wobei zu kleine und/oder zu große Polymerpartikel abgetrennt und in das Verfahren rückgeführt werden.

Die oberflächennachvernetzten Polymerpartikel können zur weiteren Verbesserung der Eigenschaften beschichtet oder nachbefeuchtet werden.

Die Nachbefeuchtung wird vorzugsweise bei 30 bis 80°C, besonders bevorzugt bei 35 bis 70°C, ganz besonders bevorzugt bei 40 bis 60°C, durchgeführt. Bei zu niedrigen Temperaturen neigen die wasserabsorbierenden Polymerpartikel zum Verklumpen und bei höheren Temperaturen verdampft bereits merklich Wasser. Die zur Nachbefeuchtung eingesetzte Wassermenge beträgt vorzugsweise von 1 bis 10 Gew.-%, besonders bevorzugt von 2 bis 8 Gew.-%, ganz besonders bevorzugt von 3 bis 5 Gew.-%, jeweils bezogen auf die wasserabsorbierenden Polymerpartikel. Durch die Nachbefeuchtung wird die mechanische Stabilität der Polymerpartikel erhöht und deren Neigung zur statischen Aufladung vermindert. Vorteilhaft wird die Nachbefeuchtung im Kühler nach der thermischen Trocknung durchgeführt.

Geeignete Beschichtungen zur Verbesserung der Anquellgeschwindigkeit sowie der Permeabilität (SFC) sind beispielsweise anorganische inerte Substanzen, wie wasserunlösliche Metallsalze, organische Polymere, kationische Polymere sowie zwei- oder mehrwertige Metallkationen. Geeignete Beschichtungen zur Staubbindung sind beispielsweise Polyole. Geeignete Beschichtungen gegen die unerwünschte Verbackungsneigung der Polymerpartikel sind beispielsweise pyrogene Kieselsäure, wie Aerosil® 200, und Tenside, wie Span® 20.

Ein weiterer Gegenstand der vorliegenden Erfindung sind die gemäß dem erfindungsgemäßen Verfahren erhältlichen wasserabsorbierenden Polymerpartikel.

Die erfindungsgemäßen wasserabsorbierenden Polymerpartikel weisen eine Zentrifugenretentionskapazität (CRC) von typischerweise mindestens 15 g/g, vorzugsweise mindestens 20 g/g, bevorzugt mindestens 25 g/g, besonders bevorzugt mindestens 30 g/g, ganz besonders bevorzugt mindestens 35 g/g, auf. Die Zentrifugenretentionskapazität (CRC) der wasserabsorbierenden Polymerpartikel beträgt üblicherweise weniger als 60 g/g.

Die erfindungsgemäßen wasserabsorbierenden Polymerpartikel weisen eine Absorption unter einem Druck von 49,2 g/cm² (AUL0.7psi) von typischerweise mindestens 10 g/g, vorzugsweise mindestens 15 g/g, bevorzugt mindestens 20 g/g, besonders bevorzugt mindestens 22 g/g, ganz besonders bevorzugt mindestens 23 g/g, auf. Die Absorption unter einem Druck von 49,2 g/cm² (AUL0.7psi) der wasserabsorbierenden Polymerpartikel beträgt üblicherweise weniger als 30 g/g.

Die erfindungsgemäßen wasserabsorbierenden Polymerpartikel weisen eine Absorption unter einem Druck von 63,0 g/cm² (AUL0.9psi) von typischerweise mindestens 5 g/g, vorzugsweise mindestens 10 g/g, bevorzugt mindestens 15 g/g, besonders bevorzugt mindestens 17 g/g, ganz besonders bevorzugt mindestens 18 g/g, auf. Die Absorption unter einem Druck von 63,0 g/cm² (AUL0.9psi) der wasserabsorbierenden Polymerpartikel beträgt üblicherweise weniger als 30 g/g.

Die erfindungsgemäßen wasserabsorbierenden Polymerpartikel weisen eine Permeabilität (SFC) von typischerweise mindestens 50 x 10⁻⁷ cm³s/g, vorzugsweise mindestens 80 x 10⁻⁷ cm³s/g, bevorzugt mindestens 100 x 10⁻⁷ cm³s/g, besonders bevorzugt mindestens 120 x 10⁻⁷ cm³s/g, ganz besonders bevorzugt mindestens 130 x 10⁻⁷ cm³s/g, auf. Permeabilität (SFC) der wasserabsorbierenden Polymerpartikel beträgt üblicherweise weniger als 250 x 10⁻⁷ cm³s/g.

Ein weiterer Gegenstand der vorliegenden Erfindung sind Hygieneartikel, enthaltend erfindungsgemäße wasserabsorbierende Polymerpartikel, insbesondere Hygieneartikel zur Damenhygiene, Hygieneartikel für leichte und schwere Inkontinenz, Windeln oder Kleintierstreu.

Die Herstellung der Hygieneartikel wird in der Monographie "Modern Superabsorbent Polymer Technology", F.L. Buchholz und A.T. Graham, Wiley-VCH, 1998, Seiten 252 bis 258, beschrieben.

Die Hygieneartikel enthalten üblicherweise eine wasserundurchlässige Rückseite, eine wasserdurchlässige Oberseite und dazwischen einen absorbierenden Kern aus den erfindungsgemäßen wasserabsorbierenden Polymerpartikeln und Fasern, vorzugsweise Cellulose. Der Anteil der erfindungsgemäßen wasserabsorbierenden Polymerpartikel im absorbierenden Kern beträgt vorzugsweise 20 bis 100 Gew.-%, bevorzugt 50 bis 100 Gew.-%.

### Methoden:

Die nachfolgend beschriebenen, mit "WSP" bezeichneten Standard-Testmethoden werden beschrieben in: "Standard Test Methods for the Nonwovens Industry", Ausgabe 2005, gemeinsam herausgegeben von den "Worldwide Strategic Partners" EDANA (Avenue Eugene Plasky, 157, 1030 Brüssel, Belgien, www.edana.org) und INDA (1100 Crescent Green, Suite 115, Cary, North Carolina 27518, U.S.A., www.inda.org). Diese Veröffentlichung ist sowohl von EDANA als auch von INDA erhältlich.

Die Messungen sollten, wenn nicht anders angegeben, bei einer Umgebungstemperatur von 23 ± 2 °C und einer relativen Luftfeuchte von 50 ± 10 % durchgeführt werden. Die wasserabsorbierenden Polymerpartikel werden vor der Messung gut durchmischt.

### Feuchtegehalt

Der Feuchtegehalt der wasserabsorbierenden Polymerpartikel wird gemäß der von der EDANA empfohlenen Testmethode Nr. WSP 230.2-05 "Mass Loss Upon Heating" bestimmt.

### Zentrifugenretentionskapazität (Centrifuge Retention Capacity)

Die Zentrifugenretentionskapazität (CRC) wird gemäß der von der EDANA empfohlenen Testmethode Nr. WSP 241.2-05 "Fluid Retention Capacity in Saline, After Centrifugation" bestimmt.

### Absorption unter einem Druck von 21,0 g/cm² (Absorption under Load)

Die Absorption unter einem Druck von 21,0 g/cm² (AUL0.3psi) der wasserabsorbierenden Polymerpartikel wird gemäß der von der EDANA empfohlenen Testmethode Nr. WSP 242.2-05 "Absorption Under Pressure, Gravimetric Determination" bestimmt.

### Extrahierbare

Der Gehalt an extrahierbaren Bestandteilen der wasserabsorbierenden Polymerpartikel wird gemäß der von der EDANA empfohlenen Testmethode Nr. WSP 270.2-05 "Extractable" bestimmt.

### Anquellgeschwindigkeit (Free Swell Rate)

Zur Bestimmung der Anquellgeschwindigkeit (FSR) werden 1,00 g (= W₁) der wasserabsorbierenden Polymerpartikel in ein 25 ml Becherglas eingewogen und gleichmäßig auf dessen Boden verteilt. Dann werden 20 ml einer 0,9 gew.-%igen Kochsalzlösung mittels eines Dispensers in ein zweites Becherglas dosiert und der Inhalt dieses Glases wird dem ersten zügig hinzugefügt und eine Stoppuhr gestartet. Sobald der letzte Tropfen Salzlösung absorbiert wird, was man am Verschwinden der Reflexion auf der Flüssigkeitsoberfläche erkennt, wird die Stoppuhr angehalten. Die genaue Flüssigkeitsmenge, die aus dem zweiten Becherglas ausgegossen und durch das Polymer im ersten Becherglas absorbiert wurde, wird durch Rückwägung des zweiten Becherglases genau bestimmt (=W₂). Die für die Absorption benötigte Zeitspanne, die mit der Stoppuhr gemessen wurde, wird als t bezeichnet. Das Verschwinden des letzten Flüssigkeitstropfens auf der Oberfläche wird als Zeitpunkt t bestimmt.

Daraus errechnet sich die Anquellgeschwindigkeit (FSR) wie folgt:

FSR [g/g s] = W2/(W₁xt)

Wenn der Feuchtegehalt der wasserabsorbierenden Polymerpartikel jedoch mehr als 3 Gew.-% beträgt, so ist das Gewicht W₁ um diesen Feuchtegehalt zu korrigieren.

### Permeabilität (Saline Flow Conductivity)

Die Permeabilität (SFC) einer gequollenen Gelschicht unter Druckbelastung von 0,3 psi (2070 Pa) wird, wie in EP 0 640 330 A1 beschrieben, als Gel-Layer-Permeability einer gequollenen Gelschicht aus wasserabsorbierenden Polymerpartikeln bestimmt, wobei die in zuvor genannter Patentanmeldung auf Seite 19 und in Figur 8 beschriebene Apparatur dahingehend modifiziert wurde, dass die Glasfritte (40) nicht mehr verwendet wird, der Stempel (39) aus gleichem Kunststoffmaterial besteht wie der Zylinder (37) und jetzt über die gesamte Auflagefläche gleichmäßig verteilt 21 gleichgroße Bohrungen enthält. Die Vorgehensweise sowie Auswertung der Messung bleibt unverändert gegenüber EP 0 640 330 A1. Der Durchfluss wird automatisch erfasst.

Die Permeabilität (SFC) wird wie folgt berechnet:

SFC [cm³s/g] = (Fg(t=0)xL0)/(dxAxWP),

wobei Fg(t=0) der Durchfluss an NaCl-Lösung in g/s ist, der anhand einer linearen Regressionsanalyse der Daten Fg(t) der Durchflussbestimmungen durch Extrapolation gegen t=0 erhalten wird, L0 die Dicke der Gelschicht in cm, d die Dichte der NaCl-Lösung in g/cm³, A die Fläche der Gelschicht in cm² und WP der hydrostatische Druck über der Gelschicht in dyn/cm².

### Oberflächenspannung des wässrigen Extraktes

Es werden 0,50 g der wasserabsorbierenden Polymerpartikel in eine kleines Becherglas eingewogen und mit 40 ml einer 0,9 gew.-%igen Salzlösung versetzt. Der Inhalt des Becherglases wird 3 Minuten bei 500 U/min mit einem Magnetrührstab gerührt, dann lässt man 2 Minuten absitzen. Schließlich wird die Oberflächenspannung (OFS) der überstehenden wässrigen Phase mit einem Digital-Tensiometer K10-ST oder vergleichbarem Gerät mit Platinplatte gemessen (Krüss GmbH, Hamburg, Deutschland). Die Messung wird bei einer Temperatur von 23°C durchgeführt.

### Beispiele

Folgende einpolymerisierbare Monomere wurden eingesetzt:

Sipomer® PAM-100 (RHODIA Operations, Aubervilliers, Frankreich), ein Polyethylenglykolmonomethacrylat Phosphatester mit einem Molekulargewicht von ca. 400 Dalton.

Sipomer® PAM-200 (RHODIA Operations, Aubervilliers, Frankreich), ein Phosphatester eines Polypropylenglykolmonomethacrylats mit einem Molekulargewicht von ca. 500 Dalton.

Sipomer® PAM-300 (RHODIA Operations, Aubervilliers, Frankreich), ein Phosphatester eines Polypropylenglykolmonoacrylats mit einem Molekulargewicht von ca. 500 Dalton.

Sipomer® PAM-4000 (RHODIA Operations, Aubervilliers, Frankreich), ein Phosphatester eines Hydroxyethylmethacrylats.

Adeka Reasoap® SR-10 (ADEKA Europe GmbH, Düsseldorf, Deutschland), das Ammoniumsalz eines Poly(oxy-1,2-ethandiyl)-α-sulfo-ω-[1-(hydroxymethyl)-2-(2-propenyloxy)ethoxy]-C₁₀/C₁₄-Alkylethers.

### Messung der Oberflächenspannung

Es wurden 1,03 g der zu untersuchenden Substanz in 1,00 I entsalztem Wasser bei 23°C gelöst. 40 ml dieser Lösung wurden in ein kleines Becherglas eingewogen. Der Inhalt des Becherglases wurde 3 Minuten bei 500 U/min mit einem Magnetrührstab gerührt. Schließlich wurde die Oberflächenspannung der überstehenden wässrigen Phase mit einem Digital-Tensiometer vom Typ K10-ST (Krüss GmbH; Hamburg; Deutschland). Die Messung wurde bei einer Temperatur von 23°C durchgeführt.

| Substanz | Oberflächenspannung [mN/m] |
|---|---|
| entsalztes Wasser | 72,0 |
| Span® 20 | 50,8 |
| Sipomer® PAM 100 | 71,2 |
| Sipomer® PAM 200 | 63,5 |
| Sipomer® PAM 300 | 60,4 |
| Sipomer® PAM 4000 | 71,2 |
| Adeka Reasoap® SR-10 | 65,0 |
| NaAMPS*) | 72,3 |
| MPEGMA**) | 70,0 |

| | |
|---|---|
| *) Natriumsalz der 2-Acrylamido-2-methylpropansulfonsäure **) Methoxypolyethylenglykol-2000-methacrylat | |

### Herstellung der Grundpolymere:

### Beispiel 1 (Vergleichsbeispiel)

Ein Kneter mit zwei Sigma-Wellen vom Modell LUK 8.0 K2 (Coperion Werner & Pfleiderer GmbH & Co. KG, Stuttgart, Deutschland) wurde zur Inertisierung mit Stickstoff durchspült und danach mit einer durch Durchperlen von Stickstoff von Sauerstoff befreiten Mischung aus 4786,99 g einer 37,3 Gew.-%igen Natriumacrylatlösung, 514,45 g Acrylsäure und 522,95 g entsalztem Wasser initial gefüllt. Anschließend wurden 6,9 g 3-fach ethoxyliertes Glycerintriacrylat (ca. 85 gew.-%ig) gelöst in 100,0 g Acrylsäure als Innenvernetzer sowie im Anschluss daran als Initiator 11,89 g einer 15 gew.-%igen wässrigen Natriumpersulfatlösung und 1,32 g einer 3 gew.-%igen wässrigen Wasserstoffperoxidlösung zugefügt. Anschließend wurden 19,82 g einer 0,5 gew.-%igen wässrigen Ascorbinsäurelösung zugegeben. Der Kneter wurde mit Geschwindigkeiten von 96 Umdrehungen pro Minute an der einen Welle und mir 48 Umdrehungen pro Minute an der anderen Welle betrieben. Unmittelbar nach Zugabe der Ascorbinsäurelösung wurde die Lösung mittels Durchleiten von Heizflüssigkeit (80°C) durch den Heizmantel des Kneters erwärmt. Sobald die Temperatur im Kneter nicht weiter anstieg wurde die Beheizung beendet und das Polymergel für weitere 13 Minuten geknetet. Anschließend wurde das Gel auf etwa 63°C gekühlt und dann aus dem Kneter entnommen. Das Gel wurde in Portionen von je 1080 g gleichmäßig auf Gitterbleche aufgeteilt und in einem Umlufttrockenschrank bei 175°C für 90 min getrocknet. Anschließend wurde das getrocknete Gel auf einem Walzenstuhl vom Modell LRC 125/70 (Bauermeister Zerkleinerungstechnik GmbH, Norderstedt, Deutschland) gemahlen, wobei nacheinander Spaltweiten von 1000 µm, 600 µm und 400 µm eingestellt wurden. Die wasserabsorbierenden Polymerpartikel wurden abgesiebt und die erhaltenen Siebfraktionen so abgemischt, dass folgende Partikelgrößenverteilung erhalten wurde:

| | |
|---|---|
| >710 µm | 0 Gew.-% |
| 600 - 710 µm | 13,3 Gew.-% |
| 500 - 600 µm | 23,3 Gew.-% |
| 300 - 500 µm | 43,6 Gew.-% |
| 150 - 300 µm | 19,8 Gew.-% |
| <150 µm | 0 Gew.-%. |

Die erhaltene Mischung wird in einem 5 I Metallgefäß in einem Röhnrad-Mischer vom Typ RRM ELTE 650 ST (J. Engelsmann AG, Ludwigshafen, Deutschland) homogenisiert.

Die erhaltenen wasserabsorbierenden Polymerpartikel wurden analysiert. Die Ergebnisse sind in Tabelle A zusammengefasst.

### Beispiel 2 (Vergleichsbeispiel)

Beispiel 1 wurde wiederholt. Es wurden zusätzlich 0,10 g Adeka Reasoap® SR-10 in der Monomerlösung gelöst.

Die erhaltenen wasserabsorbierenden Polymerpartikel wurden analysiert. Die Ergebnisse sind in Tabelle A zusammengefasst.

### Beispiel 3 (Vergleichsbeispiel)

Beispiel 1 wurde wiederholt. Es wurden zusätzlich 0,20 g Adeka Reasoap® SR-10 in der Monomerlösung gelöst.

Die erhaltenen wasserabsorbierenden Polymerpartikel wurden analysiert. Die Ergebnisse sind in Tabelle A zusammengefasst.

### Beispiel 4 (Vergleichsbeispiel)

Beispiel 1 wurde wiederholt. Es wurden zusätzlich 0,50 g Adeka Reasoap® SR-10 in der Monomerlösung gelöst.

Die erhaltenen wasserabsorbierenden Polymerpartikel wurden analysiert. Die Ergebnisse sind in Tabelle A zusammengefasst.

### Beispiel 5 (Vergleichsbeispiel)

Beispiel 1 wurde wiederholt. Es wurden zusätzlich 1,00 g Adeka Reasoap® SR-10 in der Monomerlösung gelöst.

Die erhaltenen wasserabsorbierenden Polymerpartikel wurden analysiert. Die Ergebnisse sind in Tabelle A zusammengefasst.

### Beispiel 6 (Vergleichsbeispiel)

Beispiel 1 wurde wiederholt. Es wurden zusätzlich 2,00 g Sipomer® PAM 100 in der Monomerlösung gelöst.

Die erhaltenen wasserabsorbierenden Polymerpartikel wurden analysiert. Die Ergebnisse sind in Tabelle A zusammengefasst.

### Beispiel 7

Beispiel 1 wurde wiederholt. Es wurden zusätzlich 1,00 g Sipomer® PAM 200 in der Monomerlösung gelöst.

Die erhaltenen wasserabsorbierenden Polymerpartikel wurden analysiert. Die Ergebnisse sind in Tabelle A zusammengefasst.

### Beispiel 8

Beispiel 1 wurde wiederholt. Es wurden zusätzlich 2,00 g Sipomer® PAM 200 in der Monomerlösung gelöst.

Die erhaltenen wasserabsorbierenden Polymerpartikel wurden analysiert. Die Ergebnisse sind in Tabelle A zusammengefasst.

### Beispiel 9

Beispiel 1 wurde wiederholt. Es wurden zusätzlich 3,00 g Sipomer® PAM 200 in der Monomerlösung gelöst.

Die erhaltenen wasserabsorbierenden Polymerpartikel wurden analysiert. Die Ergebnisse sind in Tabelle A zusammengefasst.

### Beispiel 10

Beispiel 1 wurde wiederholt. Es wurden zusätzlich 4,00 g Sipomer® PAM 200 in der Monomerlösung gelöst.

Die erhaltenen wasserabsorbierenden Polymerpartikel wurden analysiert. Die Ergebnisse sind in Tabelle A zusammengefasst.

### Beispiel 11

Beispiel 1 wurde wiederholt. Es wurden zusätzlich 10,00 g Sipomer® PAM 200 in der Monomerlösung gelöst.

Die erhaltenen wasserabsorbierenden Polymerpartikel wurden analysiert. Die Ergebnisse sind in Tabelle A zusammengefasst.

### Beispiel 12 (Vergleichsbeispiel)

Beispiel 1 wurde wiederholt. Es wurden zusätzlich 1,00 g Sipomer® PAM 300 in der Monomerlösung gelöst.

Die erhaltenen wasserabsorbierenden Polymerpartikel wurden analysiert. Die Ergebnisse sind in Tabelle A zusammengefasst.

### Beispiel 13 (Vergleichsbeispiel)

Beispiel 1 wurde wiederholt. Es wurden zusätzlich 2,00 g Sipomer® PAM 300 in der Monomerlösung gelöst.

Die erhaltenen wasserabsorbierenden Polymerpartikel wurden analysiert. Die Ergebnisse sind in Tabelle A zusammengefasst.

### Beispiel 14 (Vergleichsbeispiel)

Beispiel 1 wurde wiederholt. Es wurden zusätzlich 2,00 g Sipomer® PAM 4000 in der Monomerlösung gelöst.

Die erhaltenen wasserabsorbierenden Polymerpartikel wurden analysiert. Die Ergebnisse sind in Tabelle A zusammengefasst.

### Beispiel 15 (Vergleichsbeispiel)

Beispiel 1 wurde wiederholt. Es wurden zusätzlich 2,00 g Methoxypolyethylenglykol-2000-methacrylat (MPEGMA) in der Monomerlösung gelöst.

Die erhaltenen wasserabsorbierenden Polymerpartikel wurden analysiert. Die Ergebnisse sind in Tabelle A zusammengefasst.

### Beispiel 16 (Vergleichsbeispiel)

Beispiel 1 wurde wiederholt. Es wurden zusätzlich 2,00 g des Natriumsalzes der 2-Acrylamido-2-methylpropansulfonsäure (NaAMPS) in der Monomerlösung gelöst.

Die erhaltenen wasserabsorbierenden Polymerpartikel wurden analysiert. Die Ergebnisse sind in Tabelle A zusammengefasst.

### Beispiel 17 (Vergleichsbeispiel)

Beispiel 1 wurde wiederholt. Anstelle von 522,95 g entsalztem Wasser wurden nur 425,93 g entsalztes Wasser initial in den Reaktor gefüllt und 99 g einer 2 gew.-%igen wässrigen, mit Stickstoff entgasten Lösung von Sorbitanmonododecanoat (Span® 20) mit der Initiatorlösung zugegeben.

Die erhaltenen wasserabsorbierenden Polymerpartikel wurden analysiert. Die Ergebnisse sind in Tabelle A zusammengefasst.

**Tabelle A: Zusammenfassung der Grundpolymere.**

| Bsp. | Comonomer | Menge [Gew.-%] | CRC [g/g] | AUL0.3psi [g/g] | Feuchtegehalt [Gew.-%] | Extrahierbare [Gew.-%] |
|---|---|---|---|---|---|---|
| 1*) | ohne | - | 35,5 | 16,0 | 1,0 | 10,6 |
| 2*) | Adeka Reasoap® SR-10 | 0,005 | 34,9 | 20,8 | 1,3 | 9,6 |
| 3*) | Adeka Reasoap® SR-10 | 0,01 | 36,5 | 18,6 | 0,5 | 9,8 |
| 4*) | Adeka Reasoap® SR-10 | 0,025 | 33,7 | 24,1 | 1,2 | 9,4 |
| 5*) | Adeka Reasoap® SR-10 | 0,05 | 34,3 | 25,0 | 0,9 | 8,6 |
| 6*) | Sipomer® PAM 100 | 0,10 | 34,0 | 21,5 | 1,5 | 9,0 |
| 7 | Sipomer® PAM 200 | 0,05 | 35,6 | 20,1 | 0,9 | 9,4 |
| 8 | Sipomer® PAM 200 | 0,10 | 35,1 | 23,1 | 0,8 | 9,2 |
| 9 | Sipomer® PAM 200 | 0,15 | 33,6 | 25,9 | 0,8 | 8,4 |
| 10 | Sipomer® PAM 200 | 0,20 | 33,8 | 23,3 | 1,2 | 8,5 |
| 11 | Sipomer® PAM 200 | 0,50 | 32,4 | 26,9 | 1,2 | 7,9 |
| 12 | Sipomer® PAM 300 | 0,05 | 34,7 | 23,1 | 0,7 | 8,9 |
| 13 | Sipomer® PAM 300 | 0,10 | 35,2 | 17,9 | 1,6 | 8,8 |
| 14*) | Sipomer® PAM 4000 | 0,10 | 33,7 | 24,5 | 0,8 | 8,6 |
| 15*) | MPEGMA | 0,10 | 36,0 | 19,1 | 0,7 | 9,7 |
| 16*) | NaAMPS | 0,10 | 35,4 | 17,0 | 1,3 | 9,0 |
| 17*) | Span® 20**) | - | 34,4 | 19,1 | 0,7 | 8,2 |

| | | | | | | |
|---|---|---|---|---|---|---|
| *) Vergleichsbeispiel **) kein Comonomer | | | | | | |

Oberflächennachvernetzung in Gegenwart eines zusätzlichen Tensids:

### Beispiel 18 (Vergleichsbeispiel)

1200 g des Grundpolymers aus Beispiel 1 wurden zur Oberflächennachvernetzung in einem Pflugschar^{®}-Mischer mit Heizmantel vom Typ M5 (Gebr. Lödige Maschinenbau GmbH, Paderborn, Deutschland) bei 23°C und einer Wellendrehzahl von 200 Umdrehungen pro Minute mittels einer Zweistoff-Sprühdüse mit folgender Lösung beschichtet (jeweils bezogen auf das Grundpolymer):

| | |
|---|---|
| 0,992 Gew.-% | Isopropanol |
| 0,14 Gew.-% | einer Lösung aus 50 Gew.-% 1,3-Propandiol und 50 Gew.-% N-(2-Hydroxyethyl)-2-oxazolidinon |
| 0,248 Gew.-% | entsalztes Wasser |
| 0,70 Gew.-% | 1,2-Propandiol |
| 0,50 Gew.-% | einer 22 gew.-%ige wässrige Aluminiumlaktatlösung |
| 0,20 Gew.-% | einer 2 gew.-%ige wässrige Lösung von Sorbitanmonococoat |

Nach dem Aufsprühen wurde die Wellendrehzahl auf 50 Umdrehungen pro Minute reduziert und das Produkt durch Erhöhung der Temperatur des Heizmantels (Temperatur der Heizflüssigkeit 238°C) auf eine Produkttemperatur von 185°C gebracht. Dem Reaktionsgemisch wurden alle 5 Minuten in Summe 10 Proben von jeweils etwa 20 g, beginnend mit dem Erreichen der Produkttemperatur von 185°C, entnommen. Die Proben wurden jeweils auf 23°C abkühlen gelassen und bei 710 µm abgesiebt, wobei die Fraktion <710 µm verwendet wurde.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

### Beispiel 19 (Vrergleichsbeispiel)

Beispiel 18 wurde mit 1200 g des Grundpolymers aus Beispiel 3 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

### Beispiel 20 (Vergleichsbeispiel)

Beispiel 18 wurde mit 1200 g des Grundpolymers aus Beispiel 4 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

### Beispiel 21 (Vergleichsbeispiel)

Beispiel 18 wurde mit 1200 g des Grundpolymers aus Beispiel 5 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

### Beispiel 22 (Vergleichsbeispiel)

Beispiel 18 wurde mit 1200 g des Grundpolymers aus Beispiel 6 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

### Beispiel 23

Beispiel 18 wurde mit 1200 g des Grundpolymers aus Beispiel 8 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

### Beispiel 24 (V4ergleichsbeispiel)

Beispiel 18 wurde mit 1200 g des Grundpolymers aus Beispiel 13 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle B zusammengefasst.

**Tabelle B. Zusammenfassung der Polymere nach Oberflächennachvernetzung mit zusätzlichem Tensid.**

| Bsp. | Comonomer | Menge [Gew.-%] | CRC [g/g] | FSR [g/(g s)] | SFC [(cm³ s)/10⁷g)] | OFS [mN/m] |
|---|---|---|---|---|---|---|
| 18*) | ohne | - | 27,7 | 0,31 | 137 | 72,4 |
| 19*) | Adeka Reasoap® SR-10 | 0,01 | 27,5 | 0,39 | 138 | 64,2 |
| 20*) | Adeka Reasoap® SR-10 | 0,025 | 26,7 | 0,41 | 126 | 61,9 |
| 21*) | Adeka Reasoap® SR-10 | 0,05 | 26,4 | 0,51 | 127 | 56,4 |
| 22*) | Sipomer® PAM 100 | 0,10 | 27,2 | 0,34 | 152 | 72,7 |
| 23 | Sipomer® PAM 200 | 0,10 | 27,3 | 0,42 | 112 | 69,2 |
| 24*) | Sipomer® PAM 300 | 0,10 | 26,8 | 0,50 | 123 | 56,5 |

| | | | | | | |
|---|---|---|---|---|---|---|
| *) Vergleichsbeispiel | | | | | | |

Die Ergebnisse zeigen, dass die tensidischen Monomere die Anquellgeschwindigkeit (FSR) deutlich erhöhen und gleichzeitig die Oberflächenspannung senken.

Mit Sipomer® PAM 200 wurden die besten Ergebnisse erzielt, d.h. einer hohe Anquellgeschwindigkeit und nur eine geringe Senkung der Oberflächenspannung. Möglicherweise können aus dem Polymer durch Hydrolyse tensidische Gruppen abgespalten werden. In diesem Fall sind die weniger hydrolyseempfindlichen Methacrylsäureester (wie Sipomer® PAM 200) den weniger hydrolysestabilen Acrylsäureestern (wie Sipomer® PAM 300) überlegen. Weiterhin ist auch eine ausreichende Reaktivität des verwendeten Monomeren wichtig. So weisen Allylether (wie Adeka Reasorp® SR-10) eine deutlich geringere Reaktivität auf, so dass die damit hergestellten wasserabsorbierenden Polymerpartikel auch nicht umgesetztes Monomer enthalten.

Oberflächennachvernetzung in Abwesenheit eines zusätzlichen Tensids:

### Beispiel 25 (Vergleichsbeispiel)

1200 g des Grundpolymers aus Beispiel 1 wurden zur Oberflächennachvernetzung in einem Pflugschar^{®}-Mischer mit Heizmantel vom Typ M5 (Gebr. Lödige Maschinenbau GmbH, Paderborn, Deutschland) bei 23°C und einer Wellendrehzahl von 200 Umdrehungen pro Minute mittels einer Zweistoff-Sprühdüse mit folgender Lösung beschichtet (jeweils bezogen auf das Grundpolymer):

| | |
|---|---|
| 0,992 Gew.-% | Isopropanol |
| 0,14 Gew.-% | einer Lösung aus 50 Gew.-% 1,3-Propandiol und 50 Gew.-% N-(2-Hydroxyethyl)-2-oxazolidinon |
| 0,448 Gew.-% | entsalztes Wasser |
| 0,70 Gew.-% | 1,2-Propandiol |
| 0,50 Gew.-% | einer 22 gew.-%ige wässrige Aluminiumlaktatlösung |

Nach dem Aufsprühen wurde die Wellendrehzahl auf 50 Umdrehungen pro Minute reduziert und das Produkt durch Erhöhung der Temperatur des Heizmantels (Temperatur der Heizflüssigkeit 238°C) auf eine Produkttemperatur von 185°C gebracht. Dem Reaktionsgemisch wurden alle 5 Minuten in Summe 10 Proben von jeweils etwa 20 g, beginnend mit dem Erreichen der Produkttemperatur von 185°C, entnommen. Die Proben wurden jeweils auf 23°C abkühlen gelassen und bei 710 µm abgesiebt, wobei die Fraktion <710 µm verwendet wurde.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 26 (Vergleichsbeispiel)

Beispiel 25 wurde mit 1200 g des Grundpolymers aus Beispiel 2 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 27 (Vergleichsbeispiel)

Beispiel 25 wurde mit 1200 g des Grundpolymers aus Beispiel 3 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 28 (Vergleichsbeispiel)

Beispiel 25 wurde mit 1200 g des Grundpolymers aus Beispiel 5 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 29 (Vergleichsbeispiel)

Beispiel 25 wurde mit 1200 g des Grundpolymers aus Beispiel 6 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 30

Beispiel 25 wurde mit 1200 g des Grundpolymers aus Beispiel 7 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 31

Beispiel 25 wurde mit 1200 g des Grundpolymers aus Beispiel 8 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 32

Beispiel 25 wurde mit 1200 g des Grundpolymers aus Beispiel 9 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 33

Beispiel 25 wurde mit 1200 g des Grundpolymers aus Beispiel 10 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 34

Beispiel 25 wurde mit 1200 g des Grundpolymers aus Beispiel 11 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 35 (Vergleichsbeispiel)

Beispiel 25 wurde mit 1200 g des Grundpolymers aus Beispiel 14 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 36 (Vergleichsbeispiel)

Beispiel 25 wurde mit 1200 g des Grundpolymers aus Beispiel 12 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 37 (Vergleichsbeispiel)

Beispiel 25 wurde mit 1200 g des Grundpolymers aus Beispiel 13 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 38 (Vergleichsbeispiel)

Beispiel 25 wurde mit 1200 g des Grundpolymers aus Beispiel 15 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 39 (Vergleichsbeispiel)

Beispiel 25 wurde mit 1200 g des Grundpolymers aus Beispiel 16 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

### Beispiel 40 (Vergleichsbeispiel)

Beispiel 25 wurde mit 1200 g des Grundpolymers aus Beispiel 17 wiederholt.

Die erhaltenen oberflächennachvernetzten wasserabsorbierenden Polymerpartikel wurden analysiert. Die Ergebnisse der vergleichbaren Proben mit einer CRC ca. 27 g/g sind in Tabelle C zusammengefasst.

**Tabelle C. Zusammenfassung der Polymere nach Oberflächennachvernetzung ohne zusätzlichem Tensid.**

| Bsp. | Comonomer | Menge [Gew.-%] | CRC [g/g] | FSR [g/(g s)] | SFC [(cm³ s)/10⁷g]] | OFS [mN/m] |
|---|---|---|---|---|---|---|
| 25*) | ohne | - | 27,6 | 0,38 | 98 | 72,5 |
| 26*) | Adeka Reasoap® SR-10 | 0,005 | 27,2 | 0,37 | 120 | 66,5 |
| 27*) | Adeka Reasoap® SR-10 | 0,01 | 26,9 | 0,48 | 97 | 64,2 |
| 28*) | Adeka Reasoap® SR-10 | 0,05 | 26,9 | 0,54 | 104 | 57,8 |
| 29*) | Sipomer® PAM 100 | 0,10 | 27,3 | 0,38 | 74 | 72,0 |
| 30 | Sipomer® PAM 200 | 0,05 | 26,7 | 0,36 | 129 | 71,5 |
| 31 | Sipomer® PAM 200 | 0,10 | 26,9 | 0,43 | 146 | 71,0 |
| 32 | Sipomer® PAM 200 | 0,15 | 26,7 | 0,47 | 115 | 70,6 |
| 33 | Sipomer® PAM 200 | 0,20 | 27,2 | 0,42 | 87 | 69,1 |
| 34 | Sipomer® PAM 200 | 0,50 | 27,0 | 0,45 | 76 | 65,8 |
| 35*) | Sipomer® PAM 4000 | 0,10 | 27,0 | 0,38 | 93 | 72,3 |
| 36*) | Sipomer® PAM 300 | 0,05 | 27,3 | 0,50 | 96 | 64,2 |
| 37*) | Sipomer® PAM 300 | 0,10 | 26,8 | 0,52 | 165 | 57,9 |
| 38*) | MPEGMA | 0,10 | 27,7 | 0,34 | 113 | 68,5 |
| 39*) | NaAMPS | 0,10 | 27,7 | 0,39 | 103 | 72,0 |
| 40*) | Span® 20**) | - | 26,8 | 0,54 | 102 | 54,8 |

| | | | | | | |
|---|---|---|---|---|---|---|
| *) Vergleichsbeispiel **) kein Comonomer | | | | | | |

Die Ergebnisse zeigen ebenfalls, dass die tensidischen Monomere die Anquellgeschwindigkeit (FSR) deutlich erhöhen und gleichzeitig die Oberflächenspannung senken.

Die nicht erfindungsgemäßen Comonomere (wie MPEGMA und NaAMPS) haben nur einen geringen Einfluss, sowohl auf die Anquellgeschwindigkeit (FSR) als auch auf die Oberflächenspannung. Nicht einpolymerisierte Tenside (wie Span® 20) senken die Oberflächenspannung zu sehr.

## Patentansprüche

1. Verfahren zur Herstellung wasserabsorbierender Polymerpartikel durch Polymerisation einer Monomerlösung oder -suspension, enthaltend
a) mindestens ein ethylenisch ungesättigtes, säuregruppentragendes Monomer, das zumindest teilweise neutralisiert sein kann,
b) mindestens einen Vernetzer,
c) mindestens einen Initiator,
d) optional ein oder mehrere mit den unter a) genannten Monomeren copolymerisierbare ethylenisch ungesättigte Monomere und
e) optional ein oder mehrere wasserlösliche Polymere,
**dadurch gekennzeichnet, dass** die Monomerlösung oder -suspension mindestens ein ethylenisch ungesättigtes, ionisches Tensid enthält, das ethylenisch ungesättigte, ionischen Tensid eine Verbindung der allgemeinen Formel (I) 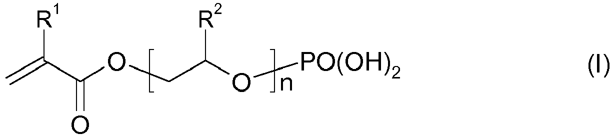 ist, wobei R¹ und R² unabhängig voneinander Methyl oder Ethyl sind und n eine ganze Zahl von 3 bis 20 bedeutet, und die erhaltenen Polymerpartikel oberflächennachvernetzt werden.

2. Verfahren gemäß Anspruch 1, **dadurch gekennzeichnet, dass** R¹ und R² Methyl sind.

3. Verfahren gemäß Anspruch 1 oder 2, **dadurch gekennzeichnet, dass** n eine ganze Zahl von 4 bis 15 bedeutet.

4. Verfahren gemäß einem der Ansprüche 1 bis 3, **dadurch gekennzeichnet, dass** n eine ganze Zahl von 5 bis 10 bedeutet.

5. Verfahren gemäß einem der Ansprüche 1 bis 4, **dadurch gekennzeichnet, dass** die Monomerlösung oder -suspension, bezogen auf das unneutralisierte Monomer a), von 0,005 bis 1 Gew.-% des ethylenisch ungesättigten, ionischen Tensids enthält.

6. Verfahren gemäß einem der Ansprüche 1 bis 6, **dadurch gekennzeichnet, dass** die Monomerlösung oder -suspension, bezogen auf das unneutralisierte Monomer a), von 0,02 bis 0,5 Gew.-% des ethylenisch ungesättigten, ionischen Tensids enthält.

7. Verfahren gemäß einem der Ansprüche 1 bis 6, **dadurch gekennzeichnet, dass** die Monomerlösung oder -suspension, bezogen auf das unneutralisierte Monomer a), von 0,05 bis 0,2 Gew.-% des ethylenisch ungesättigten, ionischen Tensids enthält.

8. Verfahren gemäß einem der Ansprüche 1 bis 7, **dadurch gekennzeichnet, dass** das Monomer a) zu mindestens 90 mol-% Acrylsäure ist.

9. Verfahren gemäß einem der Ansprüche 1 bis 8, **dadurch gekennzeichnet, dass** das Monomer a) zu 30 bis 80 mol-% neutralisiert ist.

10. Verfahren gemäß einem der Ansprüche 1 bis 9, **dadurch gekennzeichnet, dass** die Monomerlösung oder -suspension, bezogen auf das unneutralisierte Monomer a) von 0,1 bis 1 Gew.-% des Vernetzers b) enthält.

11. Wasserabsorbierende Polymerpartikel, herstellbar gemäß einem Verfahren der Ansprüche 1 bis 10.

12. Hygieneartikel, enthaltend wasserabsorbierende Polymerpartikel gemäß Anspruch 11.

## Claims

1. A process for producing water-absorbing polymer particles by polymerizing a monomer solution or suspension comprising
a) at least one ethylenically unsaturated monomer which bears acid groups and may be at least partly neutralized,
b) at least one crosslinker,
c) at least one initiator,
d) optionally one or more ethylenically unsaturated monomers copolymerizable with the monomers mentioned under a) and
e) optionally one or more water-soluble polymers,
wherein the monomer solution or suspension comprises at least one ethylenically unsaturated ionic surfactant, the ethylenically unsaturated ionic surfactant is a compound of the general formula (I) 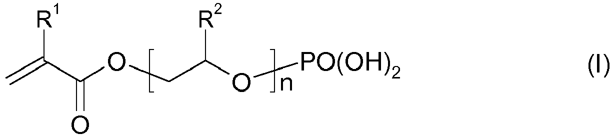 where R¹ and R² are each independently methyl or ethyl and n is an integer from 3 to 20, and the polymer particles obtained are surface postcross-linked.

2. The process according to claim 1, wherein R¹ and R² are methyl.

3. The process according to claim 1 or 2, wherein n is an integer from 4 to 15.

4. The process according to any of claims 1 to 3, wherein n is an integer from 5 to 10.

5. The process according to any of claims 1 to 4, wherein the monomer solution or suspension, based on the unneutralized monomer a), comprises from 0.005 to 1% by weight of the ethylenically unsaturated ionic surfactant.

6. The process according to any of claims 1 to 6, wherein the monomer solution or suspension, based on the unneutralized monomer a), comprises from 0.02 to 0.5% by weight of the ethylenically unsaturated ionic surfactant.

7. The process according to any of claims 1 to 6, wherein the monomer solution or suspension, based on the unneutralized monomer a), comprises from 0.05 to 0.2% by weight of the ethylenically unsaturated ionic surfactant.

8. The process according to any of claims 1 to 7, wherein monomer a) is acrylic acid neutralized to an extent of at least 90 mol%.

9. The process according to any of claims 1 to 8, wherein monomer a) has been neutralized to an extent of 30 to 80 mol%.

10. The process according to any of claims 1 to 9, wherein the monomer solution or suspension, based on the unneutralized monomer a), comprises from 0.1 to 1% by weight of the crosslinker b).

11. Water-absorbing polymer particles preparable by a process of any of claims 1 to 10.

12. A hygiene article comprising water-absorbing polymer particles according to claim 11.

## Revendications

1. Procédé pour la préparation de particules polymères absorbant l'eau par polymérisation d'une solution ou d'une suspension de monomères, contenant
a) au moins un monomère éthyléniquement insaturé, portant des groupes acides, qui peut être au moins partiellement neutralisé,
b) au moins un réticulant,
c) au moins un initiateur,
d) éventuellement un ou plusieurs monomères éthyléniquement insaturés copolymérisables avec les monomères cités en a) et
e) éventuellement un ou plusieurs polymères solubles dans l'eau,
**caractérisé en ce que** la solution ou suspension de monomères contient au moins un agent tensioactif ionique, éthyléniquement insaturé, l'agent tensioactif ionique, éthyléniquement insaturé est un composé de formule générale (I) 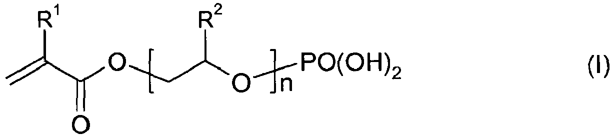 dans laquelle R¹ et R² représentent, indépendamment l'un de l'autre, méthyle ou éthyle et n signifie un nombre entier de 3 à 20, et les particules polymères obtenues sont post-réticulées en surface.

2. Procédé selon la revendication 1, **caractérisé en ce que** R¹ and R² représentent méthyle.

3. Procédé selon la revendication 1 ou 2, **caractérisé en ce que** n signifie un nombre entier de 4 à 15.

4. Procédé selon l'une quelconque des revendications 1 à 3, **caractérisé en ce que** n signifie un nombre entier de 5 à 10.

5. Procédé selon l'une quelconque des revendications 1 à 4, **caractérisé en ce que** la solution ou suspension de monomères contient, par rapport au monomère a) non neutralisé, 0,005 à 1% en poids de l'agent tensioactif ionique, éthyléniquement insaturé.

6. Procédé selon l'une quelconque des revendications 1 à 6, **caractérisé en ce que** la solution ou suspension de monomères contient, par rapport au monomère a) non neutralisé, 0,02 à 0,5% en poids de l'agent tensioactif ionique, éthyléniquement insaturé.

7. Procédé selon l'une quelconque des revendications 1 à 6, **caractérisé en ce que** la solution ou suspension de monomères contient, par rapport au monomère a) non neutralisé, 0,05 à 0,2% en poids de l'agent tensioactif ionique, éthyléniquement insaturé.

8. Procédé selon l'une quelconque des revendications 1 à 7, **caractérisé en ce que** le monomère a) est, à raison d'au moins 90% en mole, de l'acide acrylique.

9. Procédé selon l'une quelconque des revendications 1 à 8, **caractérisé en ce que** le monomère a) est neutralisé à raison de 30 à 80% en mole.

10. Procédé selon l'une quelconque des revendications 1 à 9, **caractérisé en ce que** la solution ou suspension de monomères contient, par rapport au monomère a) non neutralisé, 0,1 à 1% en poids du réticulant b).

11. Particules polymères absorbant l'eau, pouvant être préparées selon un procédé selon les revendications 1 à 10.

12. Objets hygiéniques contenant des particules polymères absorbant l'eau selon la revendication 11.
